# DEMANDE DE BREVET EUROPEEN

(11) **EP 1 504 789 A1**
(43) Date de publication de la demande: **09.02.2005**
(21) Numéro de dépôt: 04291948.0
(22) Date de dépôt: 30.07.2004
(51) Int. Cl.: A61N 1/375, H01R 4/50

(54) **Dispositif médical implantable actif comportant des moyens de fixation sans vis d'un connecteur de sonde**

(30) Priorité: 05.08.2003 FR 0309630
(71) Demandeur: ELA MEDICAL, 92541 Montrouge (FR)
(72) Inventeur: Correas, Philippe, 95220 Soisy Sous Montmorency (FR); Vanhee, Pascal, 91210 Draveil (FR)
(74) Mandataire: Dupuis-Latour, Dominique

(57) **Abrégé**

Le connecteur du générateur comporte un logement femelle axial (16) apte à recevoir un connecteur de sonde, et des moyens réversibles de retenue mécanique du connecteur de sonde dans le logement, avec un verrou rotatif (14) pourvu d'une surface de came latérale (30), définie par une section curviligne dont le rayon par rapport à l'axe de rotation est un rayon variable, et muni de moyens de couplage à un outil externe pour l'entraîner en rotation autour d'un axe (22) depuis une position escamotée, où le verrou laisse dégagé le volume intérieur du logement femelle (16) de manière à autoriser l'insertion du connecteur librement dans ce logement, jusqu'à une position de blocage, où la surface de came fait saillie à l'intérieur du logement de manière à coopérer localement avec la surface extérieure du connecteur pour exercer sur celle-ci un effort, dirigé radialement, de serrage à force du connecteur à l'intérieur du logement. Le verrou comporte deux portions semi-circulaires (32, 36) se raccordant en formant une discontinuité (42) coopérant avec une butée (44) homologue du corps du connecteur (12) définissant ladite position de blocage.

## Description

L'invention concerne les dispositifs médicaux implantables actifs. Elle sera principalement décrite dans le cas d'un stimulateur cardiaque, mais il ne s'agit là que d'un exemple de mise en oeuvre de l'invention, qui est applicable de façon beaucoup plus générale à une très grande variété de "dispositifs médicaux implantables actifs" tels que définis par la Directive 90/385/CE du 20 juin 1990 du Conseil des communautés européennes, définition qui inclut, outre les stimulateurs cardiaques, les défibrillateurs et/ou cardioverteurs, les appareils neurologiques, les pompes de diffusion de substances médicales, les implants cochléaires, les capteurs biologiques implantés, etc.

Ces dispositifs comportent un boîtier généralement désigné "générateur" ou "générateur d'impulsions" raccordé électriquement et mécaniquement à une sonde, ce raccordement étant réalisé par le chirurgien au moment de l'implantation.

On pourra à cet effet se reporter à la norme française et européenne NF EN 50077 *"Connecteur à bas profil pour stimulateurs cardiaques implantables",* qui définit un système de connexion normalisé dit "IS-1" permettant de garantir l'interchangeabilité des sondes et des générateurs d'impulsions produits par différents fabricants (l'invention n'est toutefois pas limitée au cas particulier des systèmes de connexion selon cette norme, ni même aux systèmes de connexion pour stimulateurs cardiaques).

Généralement le connecteur du générateur comprend, pour chaque borne de la connexion, une pièce métallique appelée "insert", qui est une pièce comportant un logement femelle axial recevant le connecteur de sonde à l'endroit où doit être établi le contact avec une région conductrice de ce dernier. L'insert est relié par une broche conductrice au circuit du générateur et il est noyé, en même temps que les autres inserts du connecteur, dans une matière isolante formant la "tête de connecteur" du générateur.

Le raccordement électrique et mécanique du connecteur de la sonde au connecteur du générateur est généralement réalisé au moyen d'une vis transversale dont est muni l'insert, et qui fait saillie à l'intérieur du logement recevant le connecteur de sonde. Les diverses vis sont serrées par le chirurgien au moyen d'un outil approprié (tournevis éventuellement muni d'un limiteur de couple) au moment de l'implantation.

Ce raccordement par vis présente cependant plusieurs inconvénients :
- lors des manipulations préalables à la fixation du connecteur de sonde, les vis peuvent sortir de l'insert ou, inversement, tomber dans la cavité de l'insert,
- au moment du serrage des vis, il existe un risque de déchirure par l'outil des bouchons de passage étanche (bouchons qui viennent protéger les têtes de vis du contact des fluides organiques après implantation),
- à la production, il est nécessaire de prévoir une étape spécifique de mise en place des vis dans les inserts,
- pour tenir compte de la course de la vis, il faut prévoir un dégagement suffisant en hauteur de la tête de connecteur, avec une augmentation corrélative du volume général de cette dernière.

Diverses propositions ont été formulées pour remédier à ces inconvénients, par des systèmes de raccordement des connecteurs de sonde s'affranchissant des vis de serrage, comme cela est décrit par exemple dans les. Ces systèmes de raccordement, s'ils sont efficaces du point de vue mécanique et électrique, impliquent cependant une complexité importante de structure, avec multiplication des pièces mobiles et nécessité de réaliser des pièces de forme précise et de petite dimension, ces diverses contraintes impliquant un surcoût de fabrication important.

Le EP-A-0 900 577 décrit également un système de raccordement sans vis, mais le but recherché par ce document est de disposer d'un système autorisant un raccordement rapide, sans utilisation d'aucun outil et avec une fonction "anti-retour" empêchant un retrait accidentel du connecteur de sonde. Le serrage mécanique procuré par ce système autobloquant est cependant bien moindre que celui des systèmes à vis traditionnels, ou celui des systèmes sans vis proposés par les US-A-5 951 595 ou US-A-6 080 188 précités. De plus, la nécessité de prévoir un système autobloquant implique une géométrie particulière du système pour obtenir l'effet anti-retour recherché, avec une pièce pivotante sollicitée par un ressort dans le sens opposé de l'insertion.

Les US-A-4 860 750 et US-A-4 840 580 décrivent un système de raccordement à verrou pivotant, mais ces systèmes ne permettent pas un serrage contrôlé du connecteur dans l'insert, qui peut être insuffisant avec un risque de séparation ultérieure ou de mauvais contact électrique, ou au contraire excessif avec un risque de déformation des pièces.

La présente invention a pour but de pallier les inconvénients des systèmes proposés jusqu'à présent, grâce à un système de retenue mécanique du connecteur de sonde qui soit de structure particulièrement simple et efficace et qui permette d'obtenir les mêmes performances mécaniques de serrage qu'un système à vis, mais en s'affranchissant de ces vis et donc en supprimant tous les inconvénients associés, exposés plus haut.

De plus, comme on le verra, l'invention reste parfaitement compatible avec, notamment, le standard mécanique dimensionnel IS-1, ce qui lui permet d'accepter sans modification toutes les sondes normalisées actuelles ou à venir.

Le dispositif médical implantable actif de l'invention comprend un connecteur de générateur du type enseigné notamment par les US-A-4 860 750 et US-A-4 840 580 précités, c'est-à-dire comportant un logement femelle axial apte à recevoir un connecteur de sonde, et des moyens réversibles de retenue mécanique du connecteur de sonde dans le logement, ces moyens comprenant un verrou rotatif pourvu d'une surface de came latérale et muni de moyens de couplage à un outil externe apte à exercer sur le verrou un couple d'entraînement en rotation autour d'un axe situé à distance de l'axe du logement femelle.

Cette rotation s'opère depuis une position escamotée, où le verrou laisse dégagé le volume intérieur du logement femelle de manière à autoriser l'insertion du connecteur de sonde librement dans ce logement femelle, jusqu'à une position de blocage, où la surface de came du verrou fait saillie à l'intérieur du logement femelle de manière à coopérer localement avec la surface extérieure du connecteur de sonde pour exercer sur celle-ci un effort, dirigé radialement, de serrage à force du connecteur de sonde à l'intérieur du logement femelle,

Par ailleurs, la surface de came du verrou rotatif est une surface définie par une section curviligne dont le rayon par rapport à l'axe de rotation est un rayon variable, cette section curviligne comprenant deux portions semi-circulaires, avec une portion de plus faible diamètre centrée sur l'axe de rotation du verrou rotatif, et une portion de plus fort diamètre excentrée par rapport à cet axe de rotation du verrou rotatif, les deux portions se raccordant sans solution de continuité à l'une de leurs extrémités.

De façon caractéristique de l'invention, à leur autre extrémité les deux portions semi-circulaires précitées se raccordent en formant une discontinuité coopérant avec une butée homologue du corps du connecteur définissant ladite position de blocage.

Avantageusement, les moyens de couplage à un outil externe comprennent une empreinte creuse formée axialement dans le verrou rotatif. Par ailleurs, le verrou rotatif comporte de préférence, à l'une et/ou l'autre de ses extrémités axiales, une surface cylindrique externe coopérant avec une surface cylindrique interne, de même diamètre, d'un logement du corps de connecteur recevant le verrou rotatif, pour permettre le guidage en rotation du verrou rotatif autour de son axe de pivotement.

D'autres caractéristiques de l'invention apparaîtront à la lecture de la description détaillée ci-dessous d'un exemple de réalisation.
Les figures 1 et 2 sont des vues en perspective éclatée, respectivement de dessus et de dessous, d'un insert de connecteur de générateur selon l'invention.
La figure 3 est une vue de face de cet insert.
La figure 4 est une vue en coupe de l'insert, selon IV-IV de la figure 3.
La figure 5 est une vue en coupe de l'insert, selon V-V de la figure 3, dans la position prête à recevoir un connecteur de sonde.
La figure 6 est homologue de la figure 5, avec un connecteur de sonde introduit dans l'insert et en position de serrage partiel.
La figure 7 est homologue de la figure 5, pour une position de serrage maximal.

Sur les figures, la référence 10 désigne de façon générale un dispositif de connexion pour un générateur de dispositif médical implantable actif. Ce dispositif 10 comporte une pièce massive 12 et un verrou rotatif 14, caractéristique de l'invention, qui sera décrite par la suite plus en détail.

La pièce 12 est un insert d'un connecteur de générateur d'impulsions de dispositif actif, constitué d'une pièce métallique massive électriquement reliée à l'une des bornes du générateur par une broche (non représentée) et noyée dans une résine formant la tête du connecteur du dispositif. Cet insert comprend un logement femelle axial 16 destiné à recevoir une broche de connecteur 18 (figure 6) avec laquelle l'insert sera à la fois solidarisé mécaniquement et relié électriquement.

Le verrou rotatif 14 est disposé dans un logement 20 de l'insert 12, l'axe 22 du verrou 14 et du logement 20 étant situé à distance de l'axe 24 du logement femelle 16, les deux axes 22 et 24 étant de préférence perpendiculaires entre eux.

Le verrou rotatif 14 est guidé en rotation autour de son axe 22 par un bord périphérique d'extrémité 26 coopérant avec le rebord homologue 28 de la cavité 20 à la manière d'un palier lisse.

Le verrou rotatif 14 présente en partie intermédiaire une surface de came 30 formée dans l'exemple illustré (voir notamment figure 4) d'une première surface hémicylindrique 32, dont l'axe 34 est confondu avec l'axe 22 de rotation du verrou, et d'une seconde surface hémicylindrique 36, de plus grand diamètre, dont l'axe 38 est excentré par rapport à l'axe 34. Le décalage entre les axes 34 et 38, et la différence entre les rayons des surfaces hémicylindriques 32 et 36, sont choisis de manière que ces deux surfaces se raccordent d'un côté (en 40) le long de l'une de l'une de leurs génératrices sans solution de continuité ; côté opposé, en 42, le raccordement se fait avec une discontinuité 42 susceptible de venir en appui contre une butée 44 faisant saillie à l'intérieur de la cavité 20, pour limiter la course du verrou rotatif dans le sens contraire des aiguilles d'une montre (avec les conventions de la figure 4).

Enfin, à l'extrémité axialement opposée au bord périphérique 26, le verrou rotatif 14 présente une surface cylindrique 46 d'axe 22, coopérant avec une surface homologue intérieure 48 de la cavité 20 de manière à guider également en rotation à cet endroit le verrou rotatif 14 autour de son axe 22.

Le verrou 22 comporte par ailleurs une empreinte hexagonale creuse axiale 50 permettant son entraînement en rotation par un outil approprié, de préférence un outil non spécifique tel qu'un tournevis à limiteur de couple.

La configuration initiale du dispositif de l'invention est illustrée figures 4 et 5. La figure 4 est une coupe dans un plan situé à un niveau légèrement au-dessus du plan contenant l'axe 24 du logement femelle 16 et montrant la butée 42, tandis que la coupe de la figure 5 est prise dans un plan incluant l'axe 24 du logement femelle.

Dans cette configuration initiale, le verrou rotatif est tourné dans sa position extrême dans le sens contraire des aiguilles d'une montre (avec les conventions de la figure), cette position étant définie et limitée par la discontinuité 42 venant en contact contre la butée 44. Dans cette position, c'est la partie cylindrique 32 de plus faible diamètre qui est tournée en direction du logement axial 16, et cette surface ne fait pas saillie dans le logement femelle axial 16, qui reste donc libre pour recevoir une fiche de connecteur.

La figure 6 illustre le dispositif après introduction de la broche de connecteur 18 dans le logement femelle 16. Le praticien, au moyen d'un outil introduit dans l'empreinte hexagonale 50, a commencé à faire tourner dans le sens des aiguilles d'une montre le verrou rotatif 14 par rapport à la position initiale de la figure 4.

Lorsque le mouvement de rotation est poursuivi jusqu'à ce que la génératrice 40 vienne en contact avec la surface extérieure de la broche 18, compte tenu de l'excentration entre les deux surfaces hémicylindriques de diamètres différents le verrou rotatif va faire progressivement saillie, par la surface de came 36 de plus grand diamètre, à l'intérieur du logement axial 16 du fait de l'excentration entre l'axe 38 de la surface de came hémicylindrique 36 et l'axe de rotation 22 du verrou 14.

Ceci va avoir pour effet d'exercer sur la broche de connecteur 18 un effort du verrou, dirigé radialement, qui va bloquer le connecteur à l'intérieur du logement femelle 16. L'utilisation d'un tournevis à limiteur de couple permet de calibrer de façon précise cet effort de manière à assurer une bonne solidarisation mécanique et une liaison électrique satisfaisante, sans pour autant risquer de déformer la broche du connecteur.

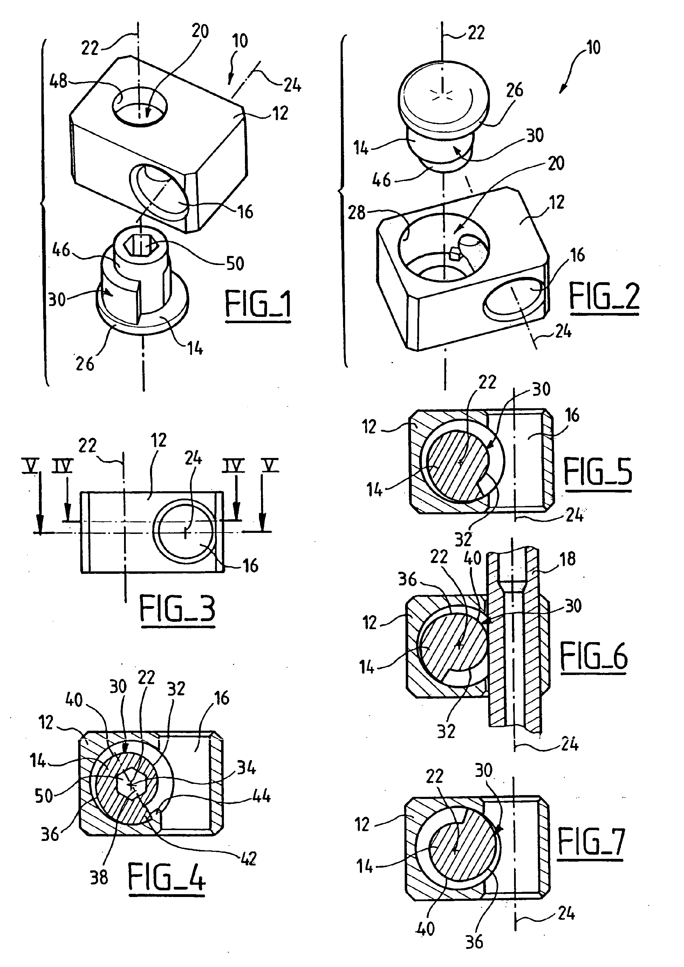

La figure 7 illustre la position extrême de rotation du verrou 14, où la saillie de la surface de came 30 à l'intérieur du logement 16 est maximale. En pratique, cette position extrême n'est pas atteinte si l'on utilise un tournevis à limiteur de couple, mais elle permet de tenir compte des jeux fonctionnels entre la broche de connecteur 18 et l'insert 12.

Les avantages du dispositif de l'invention ressortent clairement de la description que l'on vient de donner :
- rationalisation de la production : l'étape de mise en place des vis peut être supprimée, et l'insert 12 pourvu de son verrou rotatif 14 constitue un sous-ensemble complet qu'il suffit de monter dans le connecteur de la même manière qu'un insert classique, avant surmoulage de l'ensemble,
- réduction du volume du connecteur, du fait de l'absence de toute pièce saillante (plus de débordement de vis),
- l'excentrique étant noyé dans l'insert, plus de risque de déchirure du bouchon d'étanchéité par le tournevis au moment du serrage,
- plus de risque de chute de la vis dans l'insert, ou hors de l'insert,
- simplicité de mise en oeuvre pour le praticien, le verrou rotatif définissant deux positions bien précises, à savoir (i) une position pour le montage de la sonde (rotation au tournevis débrayable à gauche) et (ii) une position pour le serrage de la sonde (rotation au tournevis débrayable à droite).

Le serrage ou de desserrage de la sonde peuvent être effectués avec une rotation de course limitée, par exemple inférieure à un tour, typiquement une rotation de l'ordre d'un demi-tour à trois-quarts de tour au plus.

Par ailleurs, l'insert de l'invention permet le blocage non seulement de pièces mâles cylindriques de révolution, ce qui est le cas le plus fréquent, mais, comme on le comprendra, il peut également être appliqué le cas échéant au serrage de pièces à section polygonale.

En outre, bien que l'invention soit décrite dans le cadre d'une borne assurant simultanément la liaison électrique et le blocage mécanique, on peut dissocier ces deux fonctions, une borne selon l'invention servant au seul blocage mécanique de l'extrémité de la sonde dans le connecteur, et la liaison électrique étant assurée par un organe distinct.

## Revendications

1. Un dispositif médical implantable actif, notamment un stimulateur cardiaque, défibrillateur, cardioverteur et/ou dispositif multisite, comprenant un connecteur de générateur (10) comportant un logement femelle axial (16) apte à recevoir un connecteur de sonde (18), et des moyens réversibles de retenue mécanique du connecteur de sonde dans le logement, ces moyens comprenant un verrou rotatif (14) pourvu d'une surface de came latérale (30) et muni de moyens (50) de couplage à un outil externe apte à exercer sur le verrou un couple d'entraînement en rotation autour d'un axe (22) situé à distance de l'axe (24) du logement femelle :
- depuis une position escamotée, où le verrou laisse dégagé le volume intérieur du logement femelle de manière à autoriser l'insertion du connecteur de sonde librement dans ce logement femelle,
- jusqu'à une position de blocage, où la surface de came du verrou fait saillie à l'intérieur du logement femelle de manière à coopérer localement avec la surface extérieure du connecteur de sonde pour exercer sur celle-ci un effort, dirigé radialement, de serrage à force du connecteur de sonde à l'intérieur du logement femelle,
dans lequel la surface de came (30) du verrou rotatif (14) est une surface définie par une section curviligne dont le rayon par rapport à l'axe de rotation est un rayon variable, cette section curviligne comprenant deux portions semi-circulaires (32, 36), avec une portion de plus faible diamètre (32) centrée sur l'axe de rotation (22) du verrou rotatif, et une portion de plus fort diamètre (36) excentrée (38) par rapport à cet axe de rotation du verrou rotatif, les deux portions se raccordant sans solution de continuité à l'une de leurs extrémités (40),
dispositif **caractérisé en ce qu'**à leur autre extrémité les deux portions semi-circulaires (32, 36) se raccordent en formant une discontinuité (42) coopérant avec une butée (44) homologue du corps du connecteur (12) définissant ladite position de blocage.

2. Le dispositif de la revendication 1, dans lequel lesdits moyens de couplage à un outil externe comprennent une empreinte creuse (50) formée axialement dans le verrou rotatif (14).

3. Le dispositif de la revendication 1, dans lequel le verrou rotatif comporte, à l'une et/ou l'autre de ses extrémités axiales, une surface cylindrique externe (26, 46) coopérant avec une surface cylindrique interne (28, 48), de même diamètre, d'un logement (20) du corps de connecteur (12) recevant le verrou rotatif (14), pour permettre le guidage en rotation du verrou rotatif autour de son axe de pivotement (22).
